**Europäisches Patentamt**

**European Patent Office**

**Office européen des brevets**

(19)

(11) Veröffentlichungsnummer: **0 290 387 B2**

(12)

# NEUE EUROPÄISCHE PATENTSCHRIFT

(45) Veröffentlichungstag der neue Patentschrift: **29.11.95**

(51) Int. Cl.[6]: **C07D 211/46**, //C08K5/34

(21) Anmeldenummer: **88810266.2**

(22) Anmeldetag: **26.04.88**

Die Akte enthält technische Angaben, die nach dem Eingang der Anmeldung eingereicht wurden und die nicht in dieser Patentschrift enthalten sind.

(54) **Verfahren zur Herstellung von 4-Hydroxy-2,2,6,6-tetramethyl-piperidin.**

(30) Priorität: **05.05.87 CH 1707/87**

(43) Veröffentlichungstag der Anmeldung:
**09.11.88 Patentblatt 88/45**

(45) Bekanntmachung des Hinweises auf die Patenterteilung:
**06.03.91 Patentblatt 91/10**

(45) Bekanntmachung des Hinweises auf die Entsheidung über den Einspruch:
**29.11.95 Patentblatt 95/48**

(84) Benannte Vertragsstaaten:
**DE FR GB IT**

(56) Entgegenhaltungen:
**DE-A- 2 656 765**
**DE-A- 2 754 113**

**CHEMICAL ABSTRACTS, Band 101, Nr. 1, 2. Juli 1984, Columbus, Ohio, USA; T.G. TSAR-KOVA et al. "Electrosynthesis of 2,2,6,6-tetra-methyl-4-hydroxypiperidine" Seite 597, Spal-te 1, Zusammenfassung-Nr. 6996e**

**SRI International Report No. 85 A, Antioxi-dants" by Satish C. Nirula, November 1983**

(73) Patentinhaber: **CIBA-GEIGY AG**
**Klybeckstrasse 141**
**CH-4002 Basel (CH)**

(72) Erfinder: **Kress, Ulrich, Dr.**
**Schelmengasse 22**
**D-6140 Bensheim/Bergstrasse (DE)**
Erfinder: **Maul, Rudolf, Dr.**
**Chrodegangstrasse 6a**
**D-6143 Lorsch/Hessen (DE)**
Erfinder: **Kintopf, Siegfried, Dr.**
**Blütenweg 22**
**D-6140 Bensheim/Bergstrasse (DE)**

EP 0 290 387 B2

**Beschreibung**

Die vorliegende Erfindung betrifft ein Verfahren zur Herstellung von 4-Hydroxy-2,2,6,6-tetramethylpiperidin durch katalytische Hydrierung von 4-Oxo-2,2,6,6-tetramethylpiperidin ( = Triacetonamin).

4-Hydroxy-2,2,6,6-tetramethylpiperidin (im folgenden mit HTMP abgekürzt) ist ein grosstechnisches Zwischenprodukt, das zur Herstellung von hochwertigen Lichtstabilisatoren aus der Klasse der gehinderten Amine (2,2,6,6-Tetramethylpiperidine) benötigt wird. Es besteht grosses Interesse daran, dieses Zwischenprodukt in möglichst guter Reinheit und durch vom wirtschaftlichen und ökologischen Standpunkt optimale Verfahren herzustellen.

HTMP wird praktisch ausschliesslich durch Reduktion von Triacetonamin (im folgenden mit TAA abgekürzt) hergestellt. Es existiert eine grosse Anzahl von Veröffentlichungen, die derartige Reduktionsverfahren betreffen. So wird in J.Org.Chem. 27 (1962), 1695-1703 die Reduktion mit Natriumborhydrid und in Commis.Energ.At. (Fr.), Rapp. No. 3175, 105 pp (1967), referiert in C.A. 68 (1968), 114390m, die Reduktion mit Lithiumaluminiumhydrid beschrieben. Die vorteilhafteste und technisch brauchbarste Reduktionsmethode besteht jedoch in der katalytischen Hydrierung von TAA. Diese Hydrierung wird in Gegenwart von verschiedenen Edelmetallkatalysatoren und in einem Lösungsmittel durchgeführt, z.B. in Ethanol oder anderen Alkoholen [J.Org.Chem. 22 (1957), 1061-1065; Farmatsiya 13(3), 11-17 (1963), referiert in Heterocyclic Compounds, Vol. 37 (1964), 2881d; Helv.Chim.Acta 49 (1966), 690-695]. Die Reduktion in Alkoholen war lange Zeit die gängigste Herstellungsmethode. In der DE-A-2656763 werden tertiäre aliphatische Phosphorsäureester und in der DE-A-2656765 hydroxylgruppenfreie organische Lösungsmittel wie Kohlenwasserstoffe (z.B. Toluol, Xylol) oder Ether (z.B. Petrolether, Dibutylether, Mesitylen) als Reaktionsmedium vorgeschlagen. Schliesslich beschreibt die DE-A-2656764 und die CH-A-602644 die Hydrierung von TAA in wässrigem Medium. Letztere Methode findet bisher bei der grosstechnischen Herstellung von HTMP Verwendung.

Die Verwendung von organischen Lösungsmitteln ist mit dem Nachteil behaftet, dass aufwendige Wiedergewinnungsoperationen für das Lösungsmittel in Kauf genommen werden müssen und dass deren Verwendung vom ökologischen und prozesstechnischen Standpunkt problematisch ist. Diese Nachteile werden zwar durch das wässrige Verfahren vermieden, doch ist die erzielbare Ausbeute wegen des Auftretens von Nebenreaktionen geringer. Ausserdem stellt sich das Problem des Prozessabwassers.

Es wurde nun überraschenderweise gefunden, dass die Hydrierung von TAA mit bestimmten Katalysatoren auch ohne die Verwendung eines Lösungsmittels gelingt und dass dabei nicht nur die oben beschriebenen Nachteile verhindert werden können, sondern dass eine solche Verfahrensweise sogar weitere Vorteile mit sich bringt.

Das erfindungsgemässe Verfahren zur Herstellung von HTMP durch katalytische Hydrierung von TAA in Gegenwart eines Metallkatalysators aus der Gruppe der Ru-, Rh-, Os- und Ir-Katalysatoren oder deren Mischungen ist dadurch gekennzeichnet, dass man die Hydrierung in der Schmelze ohne Verwendung eines Lösungsmittels durchführt.

Die im erfindungsgemässen Verfahren einsetzbaren Metallkatalysatoren (Hydrierkatalysatoren) werden zweckmässig in den für diesen Verwendungszweck üblichen Formen eingesetzt, d.h. das jeweilige Metall ist auf einem Träger aufgebracht, z.B. auf Aktivkohle, Kieselgur, Aluminiumoxid, Bariumsulfat usw. Die Katalysatoren können durch weitere Metalle aktiviert werden, z.B. durch Mg, Zr oder Mo. Bevorzugt werden im erfindungsgemässen Verfahren Ru-Katalysatoren eingesetzt, z.B. Ruthenium auf Aktivkohle, Ruthenium-Moor, Ruthenium auf Kieselgur oder Ruthenium auf anderen Trägern wie Aluminiumoxid oder Bariumsulfat.

Der Katalysator ist im erfindungsgemässen Verfahren voll recyclierbar. Es ist überraschend, dass trotz Fehlens eines Lösungsmittels die Abtrennung der Produktschmelze vollständig und ohne Probleme bewerkstelligt werden kann. Wird das Verfahren diskontinuierlich (batchwise) durchgeführt, erfolgt die Abtrennung des Katalysators zweckmässig durch Filtration, wobei für diese besonders vorteilhaft Sintermetallkerzen (Sintermetallpatronen) eingesetzt werden, z.B. mit einem Porendurchmesser von 3 $\mu$m bis 25 $\mu$m. Zur weiteren Reinigung kann der Katalysator mit frischer TAA-Schmelze gespült werden, bevor er in einem nächsten Ansatz wieder eingesetzt wird.

Wird das Verfahren kontinuierlich durchgeführt, entfällt die Filtration des Katalysators. In diesem Fall wird besonders zweckmässig ein Festbett-Katalysator verwendet, z.B. eine Hochdruck-Festbetthydrieranlage. Die Produktschmelze wird in diesem Fall kontinuierlich abgezogen und mit frischer TAA-Schmelze gespeist.

Im Batchverfahren wird der Katalysator beispielsweise in einer Menge von 0,05-2%, vorzugsweise 0,1-1,5%, insbesondere 0,25-1%, bezogen auf das eingesetzte TAA, verwendet.

Die Hydrierung wird zweckmässig bei Temperaturen von 60 bis 180°C, z.B. 60 bis 150°C, vorzugsweise bei 80 bis 150°C, durchgeführt. Bei diskontinuierlicher Verfahrensweise startet man vorzugsweise die

2

Hydrierung bei tiefer Temperatur, z.B. 60°C, vorzugsweise bei 70-90°C, insbesondere bei etwa 80°C. Die Temperatur steigt dann an und man hält sie während der Hydrierung vorteilhaft konstant in einem Temperaturbereich von 100-180°C, z.B. 100-150°C, insbesondere 120-140°C, vorzugsweise 125-135°C, vor allem im Bereich von etwa 130°C. Im Fall der kontinuierlichen Arbeitsweise ist es beim Anfahren der Hydrieranlage zweckmässig, die Hydrierung bei tieferen Temperaturen zu beginnen. Im Dauerbetrieb wird in den vorstehend genannten bevorzugten Temperaturbereichen gearbeitet.

Der Wasserstoffdruck kann bei der Hydrierung beispielsweise im Bereich von 10 bis 250 bar, z.B. 10 bis 200 bar, insbesondere von 20 bis 200 bar, liegen. Welcher Wasserstoffdruck angewendet wird, hängt hauptsächlich von der zur Verfügung stehenden Hydrieranlage ab. Bei diskontinuierlicher Arbeitsweise sind Wasserstoffdrucke von 20-100 bar, z.B. 30-80 bar, oder 30-100 bar, zweckmässig. Bei Hochdruckanlagen können vorteilhaft aber auch Drucke von 100-250 bar, z.B. 150-200 bar, angewendet werden. Letztere sind vor allem bei kontinuierlicher Arbeitsweise in Hochdruck-Festbetthydrieranlagen üblich.

Die Hydrierzeit kann in weiten Grenzen schwanken, sie ist abhängig vom verwendeten Katalysator, vom Wasserstoffdruck, von der Reaktionstemperatur und der verwendeten Anlage. Sie kann z.B. von 30 Sekunden bis 2 Stunden betragen, insbesondere 1 Minute bis 1 Stunde, z.B. 1 bis 30 Minuten. Bei kontinuierlicher Arbeitsweise ist z.B. in der Praxis mit Verweilzeiten von 1 bis 60 Minuten, insbesondere von 10 bis 30 Minuten zu rechnen. Die Reaktionszeiten ebenso wie die benötigte $H_2$-Menge hängen auch von der eingesetzten TAA-Qualität, d.h. von dessen Reinheit, ab. Weniger reines TAA verlängert die Hydrierzeit und den $H_2$-Verbrauch entsprechend.

Wie bereits erwähnt, kann das erfindungsgemässe Verfahren diskontinuierlich (batchwise) oder kontinuierlich durchgeführt werden. Bevorzugte Reaktionsbedingungen für diese beiden Verfahrensweisen sind oben angegeben. Im Fall der kontinuierlichen Arbeitsweise wird besonders zweckmässig ein Katalysator-Festbett verwendet.

Hydrierungen in der Schmelze sind bei anderen Reaktionstypen im Prinzip bekannt, z.B. die Hydrierung von Nitroverbindungen, Carbonsäuren usw. Siehe EP-A-2308, EP-A-805, US-A-4,076,946 und Kinet.Katal. 1984, 25 (3), 578-82, referiert in C.A. 101 (1984), 90186w. Es konnte jedoch nicht erwartet werden, dass die Hydrierung von TAA, die seit langer Zeit Gegenstand von intensiven Versuchen zur Verbesserung des Verfahrens war, in der Schmelze glatt gelingt, dass viele Nachteile der bekannten Reduktionsverfahren vermieden werden können und dass sogar durch die Verfahrensweise in der Schmelze bedeutende Vorteile erreicht werden können.

Unter den besonderen Vorteilen des erfindungsgemässen Verfahrens gegenüber den bekannten Lösungsmittelverfahren seien - abgesehen von der Vermeidung von Lösungsmittelregenerations- und Abwasserproblemen - beispielsweise die folgenden erwähnt: Wesentlich erhöhte Raum-Zeit-Ausbeute, kürzere Reaktionszeiten, weniger Nebenreaktionen, sehr reines Produkt, hohe Absolutausbeuten, erhöhte Selektivität, keine zusätzlichen Reinigungsoperationen vor Weiterverwendung des erhaltenen HTMP.

Der letztgenannte Vorteil ist zum Beispiel wesentlich, wenn das HTMP für die Herstellung von 1-(2-Hydroxyethyl)-4-hydroxy-2,2,6,6-tetramethylpiperidin (HE-HTMP) verwendet wird. Für diesen Zweck wird HTMP mit Ethylenoxid umgesetzt. Die sonst nötige Zwischenisolierung bzw. Reinigung des HTMP entfällt, wenn nach der erfindungsgemässen Schmelzhydrierung von TAA gearbeitet wird. Für die Herstellung von HE-HTMP kann vorteilhaft auch das in der europäischen Patentanmeldung Nr. 86810554.5 beschriebene Verfahren verwendet werden, wobei die Einengung der HTMP-Rohlösung entfällt, da nach dem erfindungsgemässen Verfahren bereits eine reine HTMP-Schmelze erhalten wird.

Die nachfolgenden Beispiele erläutern das erfindungsgemässe Verfahren weiter. Darin beziehen sich, ebenso wie in der übrigen Beschreibung, Teile- und Prozentangaben auf das Gewicht, sofern nichts anderes angegeben ist.

Beispiele 1-5

100 g destilliertes TAA (Reinheit: 99%) und 1 g 5%iges Ruthenium auf Aktivkohle (Ru/C) werden in einen 300 ml Hydrierautoklaven gefüllt. Nach Spülung der Apparatur mit $H_2$ wird der gewünschte Wasserstoffdruck eingestellt und die Hydrierung bei 80°C gestartet. Die Temperatur steigt schnell auf 130°C und wird dann konstant gehalten. Nach Beendigung der $H_2$-Aufnahme wird der Katalysator bei 150°C über eine Sintermetallpatrone (Porengrösse 7-15 μm) nach dem Bodenventil filtriert. Der Katalysator wird, gegebenenfalls nach Spülung mit reiner TAA-Schmelze, für den nächsten Ansatz wiederverwendet. Die filtrierte HTMP-Schmelze wird ausgewogen und gaschromatographisch analysiert. In der nachfolgenden Tabelle sind für einige Ansätze bei verschiedenem $H_2$-Druck die erhaltenen Analysenwerte, die Wasserstoffaufnahme und die Hydrierzeit angegeben.

Tabelle

| Beispiel | H₂-Druck (bar) | Hydrierzeit (min.) | H₂-Aufnahme (% d.Th) | TAA-Umsatz zu HTMP (in Gew.%) | HTMP in der Schmelze (in Gew.%) |
|---|---|---|---|---|---|
| 1 | 20 | 28 | 100 | 99,6 | 98,2 |
| 2 | 40 | 15 | 110 | 96,4 | 96,7 |
| 3 | 100 | 7 | 101 | 99,9 | >99 |
| 4 | 100 | 2 | 99 | 99,9 | >99 |
| 5 | 200 | 1 | 100 | 99,9 | >99 |

Beispiel 6

Kontinuierliche Hydrierung:

In eine Hochdruck-Festbetthydrieranlage (Volumen: 500 ml) enthaltend 150 ml Ru/C-Katalysator-Schüttung wird mittels einer beheizten Pumpe TAA-Schmelze bei 70-80°C eingepumpt. Die Gesamtapparatur steht unter einem H₂-Druck von 150-200 bar und wird bei einer Temperatur von 130°C gehalten. Die HTMP-Schmelze verlässt nach einer Verweilzeit von 10 bis 30 Minuten den Reaktor und wird gaschromatographisch analysiert. Es wird ein TAA-Umsatz zu HTMP von etwa 99,9 Gew.-% erzielt, der Gehalt der Produktschmelze an HTMP ist >99%.

**Patentansprüche**

1. Verfahren zur Herstellung von 4-Hydroxy-2,2,6,6-tetramethylpiperidindurch katalytische Hydrierung von 4-Oxo-2,2,6,6-tetramethylpiperidin in Gegenwart eines Metallkatalysators aus der Gruppe der Ru-, Rh-, Os- und Ir-Katalysatoren oder deren Mischungen, dadurch gekennzeichnet, dass man die Hydrierung in der Schmelze ohne Verwendung eines Lösungsmittels durchführt.

2. Verfahren nach Anspruch 1, worin man als Katalysator einen Ru-Katalysator verwendet.

3. Verfahren nach Anspruch 1 oder 2, worin man bei Temperaturen von 60 bis 180°C hydriert.

4. Verfahren nach einem der Ansprüche 1 bis 3, worin man bei einem Wasserstoffdruck von 10 bis 250 bar hydriert.

5. Verfahren nach einem der Ansprüche 1 bis 4, dadurch gekennzeichnet, dass man es diskontinuierlich (batchwise) durchführt.

6. Verfahren nach Anspruch 5, worin bei einem Wasserstoffdruck von 30-100 bar hydriert wird.

7. Verfahren nach einem der Ansprüche 1 bis 4, dadurch gekennzeichnet, dass man es kontinuierlich durchführt.

8. Verfahren nach Anspruch 7, worin der Katalysator als Festbett vorliegt.

9. Verfahren nach Anspruch 7 oder 8, worin bei einem Wasserstoffdruck von 150-200 bar hydriert wird.

**Claims**

1. A process for the preparation of 4-hydroxy-2,2,6,6-tetramethylpiperidine by catalytic hydrogenation of 4-oxo-2,2,6,6-tetramethylpiperidine in the presence of a metal catalyst from the group consisting of Ru, Rh, Os and Ir catalysts or mixtures thereof, which comprises carrying out the hydrogenation in the melt without using a solvent.

2. The process according to claim 1, wherein an Ru catalyst is used as the catalyst.

3. The process according to claim 1 or 2, wherein the hydrogenation is carried out at temperatures from 60 to 180°C.

4. The process according to any one of claims 1 to 3, wherein the hydrogenation is carried out under a hydrogen pressure of 10 to 250 bar.

5. The process according to any one of claims 1 to 4, wherein a batchwise procedure is followed.

6. The process according to claim 5, wherein the hydrogenation is carried out under a hydrogen pressure of 30-100 bar.

7. The process according to any one of claims 1 to 4, wherein the procedure is continuous.

8. The process according to claim 7, wherein the catalyst is in the form of a fixed bed.

9. The process according to either of claims 7 and 8, wherein the hydrogenation is carried out under a hydrogen pressure of 150-200 bar.

**Revendications**

1. Procédé pour préparer l'hydroxy-4 tétraméthyl-2,2,4,6 pipéridine par hydrogénation catalytique de l'oxo-4 tétraméthyl-2,2,6,6 pipéridine en présence d'un catalyseur pris dans l'ensemble constitué par les catalyseurs au ruthénium, au rhodium, à l'osmium et à l'iridium et leurs mélanges, procédé caractérisé en ce qu'on effectue l'hydrogénation à l'état fondu sans utiliser de solvant.

2. Procédé selon la revendication 1, dans lequel on utilise, comme catalyseur, un catalyseur au ruthénium.

3. Procédé selon l'une des revendications 1 et 2, dans lequel on hydrogène à des températures de 60 à 180°C.

4. Procédé selon l'une quelconque des revendications 1 à 3, dans lequel on hydrogène sous une pression d'hydrogène de 10 à 250 bar.

5. Procédé selon l'une quelconque des revendications 1 à 4, caractérisé en ce qu'il est effectué en discontinu.

6. Procédé selon la revendication 5 dans lequel on hydrogène sous une pression d'hydrogène de 30 à 100 bar.

7. Procédé selon l'une quelconque des revendications 1 à 4, caractérisé en ce qu'il est effectué en continu.

8. Procédé selon la revendication 7 dans lequel le catalyseur est sous la forme d'un lit fixe.

9. Procédé selon l'une des revendications 7 et 8, dans lequel on hydrogène sous une pression d'hydrogène de 150 à 200 bar.